# EUROPÄISCHE PATENTANMELDUNG

(11) **EP 4 817 526 A1**
(43) Veröffentlichungstag der Anmeldung: **07.10.2026**
(21) Anmeldenummer: 26159918.7
(22) Anmeldetag: 20.02.2026
(51) Int. Cl.: A61B 6/03, A61B 6/10

(54) **VERKLEIDUNGSTEIL FÜR EINE GANTRY EINES COMPUTERTOMOGRAPHIEGERÄTS**

(30) Priorität: 31.03.2025 DE 202025101738 U
(71) Anmelder: Siemens Healthineers AG, 91301 Forchheim (DE)
(72) Erfinder: Eichner, Christoph, 91301 Forchheim (DE); Fehre, Jens, 91301 Forchheim (DE)
(74) Vertreter: Siemens Healthineers Patent Attorneys

(57) **Zusammenfassung**

Die Erfindung betrifft ein Verkleidungsteil für eine Gantry eines Computertomographiegeräts,
- wobei das Verkleidungsteil ein Ringscheiben-Formteil und ein Zylindermantel-Formteil aufweist,
- wobei das Ringscheiben-Formteil und das Zylindermantel-Formteil derart koaxial zu einer Systemachse angeordnet sind, dass ein Innenumfang des Ringscheiben-Formteils an eine Basis des Zylindermantel-Formteils angefügt ist,
- wobei das Ringscheiben-Formteil einen ersten Wandungsbereich und einen zweiten Wandungsbereich aufweist, die in Bezug auf die Systemachse einander gegenüberliegen, an den Innenumfang des Ringscheiben-Formteils angrenzen und sich im Wesentlichen senkrecht zu der Systemachse flächig erstrecken,
- wobei das Ringscheiben-Formteil eine Versteifungsstruktur aufweist, welche sich derart im Wesentlichen parallel zu der Systemachse flächig von dem Zylindermantel-Formteil weg erstreckt, dass sich der erste Wandungsbereich und der zweite Wandungsbereich in Bezug auf die Systemachse zwischen der Versteifungsstruktur und dem Zylindermantel-Formteil befinden.

## Beschreibung

Die Erfindung betrifft ein Verkleidungsteil für eine Gantry eines Computertomographiegeräts. Die Erfindung betrifft ferner eine Gantry und ein Computertomographiegerät.

Verkleidungsteile einer Gantry eines Computertomographiegeräts sollten einerseits ein geringes Gewicht und einen geringen Bauraumbedarf haben, andererseits sollten sie möglichst stabil sein, insbesondere um vorgegebene Sicherheitsabstände zu rotierenden Komponenten der Gantry auch unter Belastung einzuhalten.

Als Stand der Technik sind hierbei die DE 103 34 326 A1, DE 10 2007 010 061 A1, DE 10 2016 208 328 A1, DE 20 2021 103 613 U1 und DE 20 2024 104 403 U1 zu nennen.

Die Erfindung hat die Aufgabe, eine Alternative zu herkömmlichen Gantry-Verkleidungsteilen bei Computertomographiegeräten bereitzustellen, insbesondere im Hinblick auf die vorgenannten Anforderungen. Die unabhängigen Ansprüche betreffen Lösungen dieser Aufgabe. Die abhängigen Ansprüche betreffen besondere Ausführungsformen dieser Lösungen. Unabhängig vom grammatikalischen Geschlecht eines bestimmten Begriffes sind Personen mit männlicher, weiblicher oder anderer Geschlechteridentität mit umfasst.

Die Erfindung betrifft ein Verkleidungsteil für eine Gantry eines Computertomographiegeräts,
- wobei das Verkleidungsteil ein Ringscheiben-Formteil und ein Zylindermantel-Formteil aufweist,
- wobei das Ringscheiben-Formteil und das Zylindermantel-Formteil derart koaxial zu einer Systemachse angeordnet sind, dass ein Innenumfang des Ringscheiben-Formteils an eine Basis des Zylindermantel-Formteils angefügt ist,
- wobei das Ringscheiben-Formteil einen ersten Wandungsbereich und einen zweiten Wandungsbereich aufweist, die in Bezug auf die Systemachse einander gegenüberliegen, an den Innenumfang des Ringscheiben-Formteils angrenzen und sich im Wesentlichen senkrecht zu der Systemachse flächig erstrecken,
- wobei das Ringscheiben-Formteil eine Versteifungsstruktur aufweist, welche sich derart im Wesentlichen parallel zu der Systemachse flächig von dem Zylindermantel-Formteil weg erstreckt, dass sich der erste Wandungsbereich und der zweite Wandungsbereich in Bezug auf die Systemachse zwischen der Versteifungsstruktur und dem Zylindermantel-Formteil befinden.

Ferner wird hiermit ein Verkleidungsteil für eine Gantry eines Computertomographiegeräts offenbart,
- wobei das Verkleidungsteil ein Ringscheiben-Formteil und ein Zylindermantel-Formteil aufweist,
- wobei das Ringscheiben-Formteil und das Zylindermantel-Formteil derart koaxial zu einer Systemachse angeordnet sind, dass ein Innenumfang des Ringscheiben-Formteils an eine Basis des Zylindermantel-Formteils angefügt ist,
- wobei das Ringscheiben-Formteil einen ersten Wandungsbereich und einen zweiten Wandungsbereich aufweist, die in Bezug auf die Systemachse einander gegenüberliegen, an den Innenumfang des Ringscheiben-Formteils angrenzen und sich im Wesentlichen senkrecht zu der Systemachse flächig erstrecken,
- wobei das Ringscheiben-Formteil eine Versteifungsstruktur aufweist.

Insbesondere kann vorgesehen sein, dass die Versteifungsstruktur sich im Wesentlichen parallel zu der Systemachse flächig von dem Zylindermantel-Formteil weg erstreckt und/oder dass sich der erste Wandungsbereich und der zweite Wandungsbereich in Bezug auf die Systemachse zwischen der Versteifungsstruktur und dem Zylindermantel-Formteil befinden.

Insbesondere kann vorgesehen sein, dass die Versteifungsstruktur sich derart im Wesentlichen parallel zu der Systemachse flächig von dem Zylindermantel-Formteil weg erstreckt, dass sich der erste Wandungsbereich und der zweite Wandungsbereich in Bezug auf die Systemachse zwischen der Versteifungsstruktur und dem Zylindermantel-Formteil befinden.

Der Innenumfang des Ringscheiben-Formteils und die Basis des Zylindermantel-Formteils können beispielsweise aneinandergeklebt sein. Die Wandstärke des Ringscheiben-Formteils und/oder des Zylindermantel-Formteils kann beispielsweise kleiner als 30 mm, insbesondere kleiner als 20 mm, sein. Insbesondere kann vorgesehen sein, dass der erste Wandungsbereich, der zweite Wandungsbereich und die Versteifungsstruktur einstückig ineinander übergehen.

Die Versteifungsstruktur bewirkt eine erhöhte Steifigkeit des Verkleidungsteils. Damit kann ein Sicherheitsabstand zwischen dem Ringscheiben-Formteil und einer Trajektorie von rotierenden Komponenten der Gantry verringert werden. Damit kann bei einem Kopf-Computertomographiegerät ein Patient näher an die Scanebene herangeführt werden und somit die Scantiefe erhöht werden. Durch eine hohe Scantiefe kann mehr vom Kopf- und Halsbereich des Patienten gescannt werden, wodurch der mögliche Untersuchungsbereich erhöht wird.

Die Scantiefe kann erhöht werden, indem das Verkleidungsteil näher an die Strahlengeometrie gebracht wird. Dabei kann zwischen dem rotierenden Teil der Gantry, worin sich die Strahlengeometrie befindet, und dem Verkleidungsteil ein Sicherheitsabstand eingehalten werden, damit das Verkleidungsteil nicht durch im klinischen Arbeitsablauf auftretende Belastungen in die rotierenden Teile der Gantry gedrückt werden kann.

Durch die erhöhte Steifigkeit kann die Menge an Material und somit das Gesamtgewicht des Verkleidungsteils beispielsweise so weit reduziert werden, dass ein manuelles Entfernen des Verkleidungsteils für Wartungszwecke von einer Person durchgeführt werden kann.

Eine Ausführungsform sieht vor, dass die Versteifungsstruktur eine erste Teilstruktur aufweist, wobei sich die erste Teilstruktur in einer ersten Versteifungsebene, die zu der Systemachse im Wesentlichen parallel ist, flächig erstreckt. Insbesondere kann vorgesehen sein, dass die erste Teilstruktur an den Innenumfang des Ringscheiben-Formteils angrenzt und/oder dass die erste Versteifungsebene im Wesentlichen tangential zu einer Mantelfläche des Zylindermantel-Formteils verläuft.

Eine Ausführungsform sieht vor, dass die Versteifungsstruktur eine zweite Teilstruktur aufweist, wobei sich die zweite Teilstruktur in einer zweiten Versteifungsebene, die zu der ersten Versteifungsebene parallel ist, flächig erstreckt, wobei sich der erste Wandungsbereich und der zweite Wandungsbereich in Bezug auf eine Richtung, die zu der Systemachse senkrecht ist, zwischen der ersten Versteifungsebene und der zweiten Versteifungsebene befinden.

Eine Ausführungsform sieht vor, dass das Ringscheiben-Formteil und/oder das Zylindermantel-Formteil aus einem faserverstärkten Verbundstoff, insbesondere einem carbonfaserverstärkten Verbundstoff, hergestellt ist.

Die Formteile können beispielsweise durch ein Autoklav-Verfahren und/oder ein Tiefziehverfahren hergestellt sein. Aus Faserverbundstoffen können Verkleidungsteile mit einer hohen Steifigkeit hergestellt werden, sodass der Sicherheitsabstand zu der Trajektorie der rotierenden Komponenten verringert werden kann und somit das Verkleidungsteil näher an die Strahlengeometrie gerückt werden kann. Damit kann die Scantiefe vergrößert werden. Des Weiteren kann durch die Verwendung von Faserverbundstoffen die Wandstärke des Verkleidungsteils stark verringert werden. Dadurch kann das Cover noch näher an die Strahlengeometrie gebracht werden, was eine weitere Vergrößerung der Scantiefe bewirkt.

Die Erfindung betrifft ferner eine Gantry für ein Computertomographiegerät, wobei die Gantry eine Verkleidung zum Abgrenzen eines Innenbereichs der Gantry von einer Umgebung der Gantry aufweist, wobei die Verkleidung das erfindungsgemäße Verkleidungsteil aufweist, wobei die Gantry eine Öffnung aufweist, wobei ein Untersuchungsobjekt entlang der Systemachse in die Öffnung einführbar ist, wobei das Zylindermantel-Formteil den Innenbereich der Gantry von der Öffnung abgrenzt.

Eine Ausführungsform sieht vor, dass ein Strahlenschutzmaterial an einer dem Innenbereich der Gantry zugewandten Seite des Ringscheiben-Formteils flächig anliegt. Das Strahlenschutzmaterial kann beispielsweise in Form einer Folie, insbesondere einer Bleifolie, ausgebildet sein und/oder einem stufenförmigen Verlauf des Verkleidungsteils folgen. Durch das Strahlenschutzmaterial kann die Umgebung der Gantry, in der sich beispielsweise eine Bedienperson oder ein Patient befinden können, vor Streustrahlung geschützt werden. Das relativ hohe Gewicht des Strahlenschutzmaterials kann durch ein relativ leichtes Gewicht des Verkleidungsteils teilweise ausgeglichen werden.

Eine Ausführungsform sieht vor, dass die Gantry ein Projektionsdatenakquisitionssystem aufweist, wobei das Projektionsdatenakquisitionssystem zum Erfassen von Projektionsdaten des in die Öffnung eingeführten Untersuchungsobjekts eingerichtet ist, wobei ein Abstand der Versteifungsstruktur zu der Systemachse, insbesondere ein Abstand der ersten Versteifungsebene und/oder der zweiten Versteifungsebene zu der Systemachse, kleiner ist als ein Abstand des Projektionsdatenakquisitionssystems zu der Systemachse.

Das Untersuchungsobjekt kann insbesondere ein Kopf eines Patienten sein. Die Schultern des Patienten können beispielsweise breiter als die Öffnung sein. Insbesondere kann ein Formschluss der Schultern des Patienten mit dem ersten Wandungsbereich und dem zweiten Wandungsbereich einem weiteren Einführen des Kopfes des Patienten entlang der Systemachse in die Öffnung formschlüssig entgegenwirken.

Der Abstand des Projektionsdatenakquisitionssystems von der Systemachse kann beispielsweise ein Abstand einer Röntgenquelle des Projektionsdatenakquisitionssystems und/oder ein Abstand eines Röntgendetektors des Projektionsdatenakquisitionssystems von der Systemachse sein.

Eine Ausführungsform sieht vor, dass die Versteifungsstruktur ein Fenster derart aufweist, dass eine optische Achse einer optischen Komponente des Computertomographiesystems durch das Fenster dringen kann, wenn sich die optische Komponente in dem Innenbereich der Gantry befindet.

Die optische Komponente kann beispielsweise eine Kamera zur Beobachtung des Untersuchungsobjekts und/oder des Patienten und/oder eine Lasereinheit zur laserbasierten Positionierung des Untersuchungsobjekts sein. Insbesondere kann vorgesehen sein, dass sich der erste Wandungsbereich und der zweite Wandungsbereich in Bezug auf die Systemachse zwischen der optischen Komponente und dem Zylindermantel-Formteil befinden. Insbesondere kann vorgesehen sein, dass die optische Achse im Wesentlichen senkrecht zu der Systemachse ist und/oder dass sich der erste Wandungsbereich und der zweite Wandungsbereich in Bezug auf die Systemachse zwischen der optischen Achse und dem Zylindermantel-Formteil befinden.

Eine Ausführungsform sieht vor, dass das Verkleidungsteil eine hutzenartige Wölbung hin zu der Umgebung der Gantry aufweist, wobei sich die hutzenartige Wölbung zu der Versteifungsstruktur, insbesondere zu der ersten Teilstruktur der Versteifungsstruktur, hin aufweitet, wobei ein Teilbereich des Innenbereichs der Gantry durch die hutzenartige Wölbung und die Versteifungsstruktur von der Umgebung der Gantry abgegrenzt ist, wobei sich in dem Teilbereich des Innenbereichs der Gantry eine Halterung zur Befestigung der optischen Komponente an der Versteifungsstruktur befindet.

Die Erfindung betrifft ferner ein Computertomographiegerät, insbesondere ein mobiles Kopf-Computertomographiegerät, aufweisend eine erfindungsgemäße Gantry.

Eine Ausführungsform sieht vor, dass die Gantry einen ersten Gantryteil, einen zweiten Gantryteil und einen dritten Gantryteil aufweist, wobei der erste Gantryteil einen drehbar gelagerten Rotor mit einem Projektionsdatenakquisitionssystem aufweist, wobei der dritte Gantryteil zumindest einen Abschnitt der Öffnung aufweist, wobei die Haltestruktur mittels der Schwenkvorrichtung mit dem dritten Gantryteil verbunden und relativ zu dem dritten Gantryteil um die Schwenkachse schwenkbar gelagert ist. Das Projektionsdatenakquisitionssystem kann beispielsweise eine Röntgenquelle und einen Röntgendetektor, welcher mit der Röntgenquelle zusammenwirkt, aufweisen.

Dabei kann der erste Gantryteil relativ zu dem zweiten Gantryteil und relativ zu dem dritten Gantryteil derart bewegbar gelagert sein, dass eine Translationsbewegung des ersten Gantryteils relativ zu dem zweiten Gantryteil und relativ zu dem dritten Gantryteil ausgeführt werden kann, während gleichzeitig dazu der zweite Gantryteil und der dritte Gantryteil relativ zu dem Untersuchungsobjekt ruhen und die Strahlenschutzvorrichtung relativ zu dem Untersuchungsobjekt und relativ zu dem zumindest einen Abschnitt der Öffnung ruht, wenn sich das Untersuchungsobjekt in der Öffnung befindet.

Das Untersuchungsobjekt kann beispielsweise ein Körperteil eines Patienten, insbesondere ein Kopf eines Patienten, sein. Das Computertomographiegerät kann insbesondere als Kopf-Computertomographiegerät und/oder als mobiles Computertomographiegerät ausgebildet sein. Bei dem Patienten kann es sich beispielsweise um einen Menschen, insbesondere um ein Baby, oder um ein Tier handeln. Eine Längsachse des Patienten kann beispielsweise zu der Systemachse parallel, insbesondere identisch, oder windschief sein oder die Systemachse schneiden. Das Untersuchungsobjekt kann beispielsweise ein Gegenstand, insbesondere ein Phantom zur Kalibrierung des Computertomographiegeräts, sein.

Im Rahmen der Erfindung können Merkmale, welche in Bezug auf unterschiedliche Ausführungsformen der Erfindung und/oder unterschiedliche Anspruchskategorien (Verfahren, Verwendung, Vorrichtung, System, Anordnung usw.) beschrieben sind, zu weiteren Ausführungsformen der Erfindung kombiniert werden. Beispielsweise kann ein Anspruch, der eine Vorrichtung betrifft, auch mit Merkmalen, die im Zusammenhang mit einem Verfahren beschrieben oder beansprucht sind, weitergebildet werden und umgekehrt. Funktionale Merkmale eines Verfahrens können dabei durch entsprechend ausgebildete gegenständliche Komponenten ausgeführt werden. Die Verwendung des unbestimmten Artikels "ein" bzw. "eine" schließt nicht aus, dass das betroffene Merkmal auch mehrfach vorhanden sein kann.

Im Folgenden werden Merkmale der Erfindung anhand von Beispielen unter Hinweis auf die beigefügten Figuren erläutert. Die Darstellung in den Figuren ist schematisch, stark vereinfacht und nicht zwingend maßstabsgetreu.

Die Figuren 1 bis 4 zeigen ein Verkleidungsteil für eine Gantry eines Computertomographiegeräts in verschiedenen Ansichten.

Die Figuren 5 und 6 zeigen ein Computertomographiegerät mit einer Gantry.

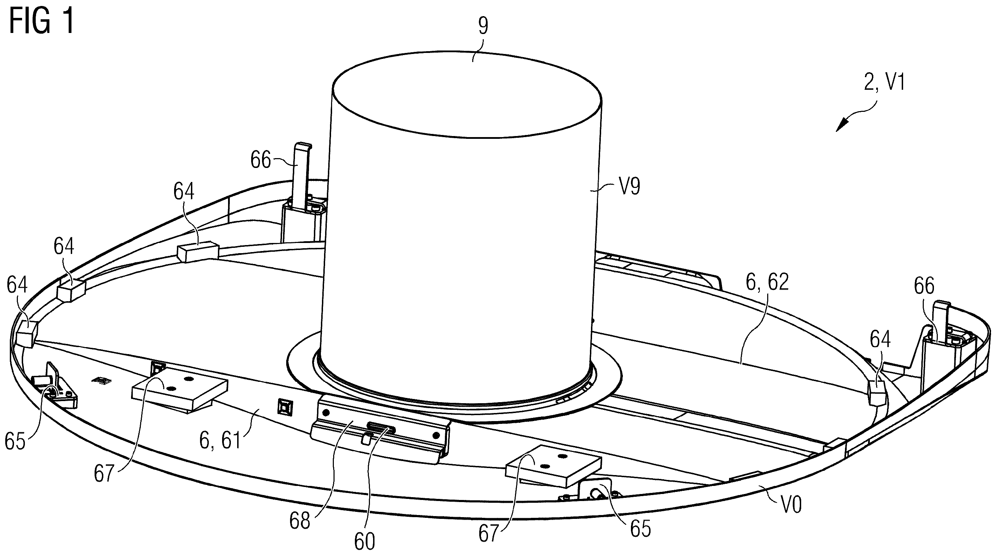

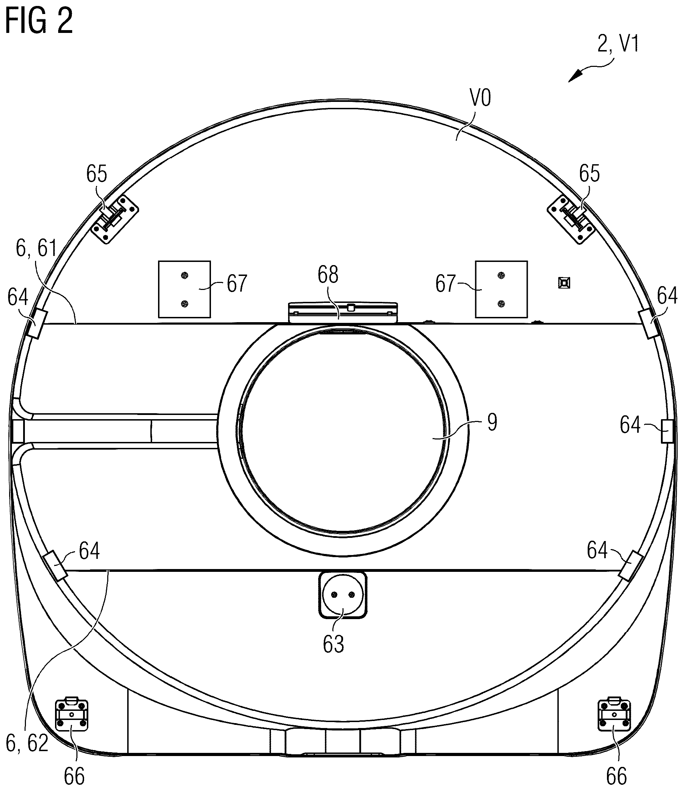

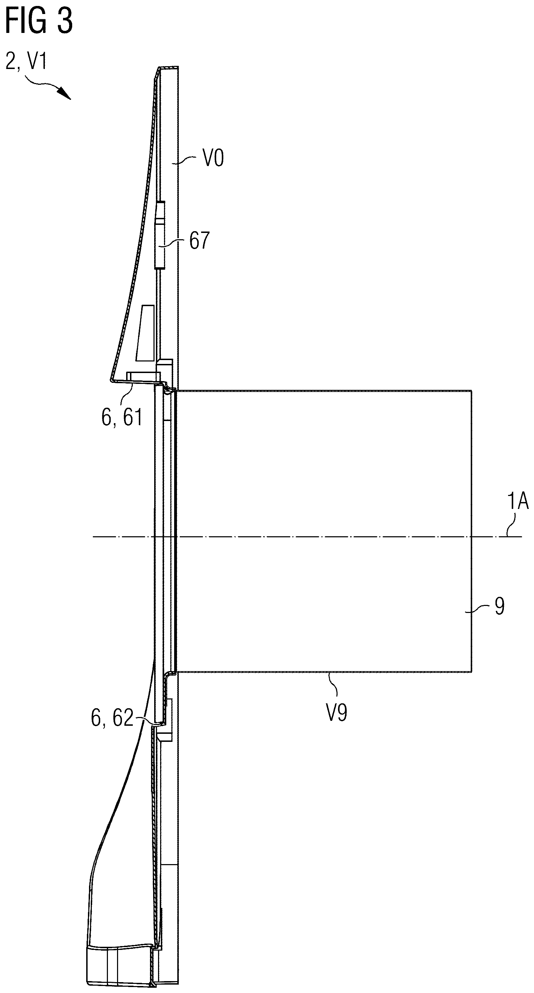

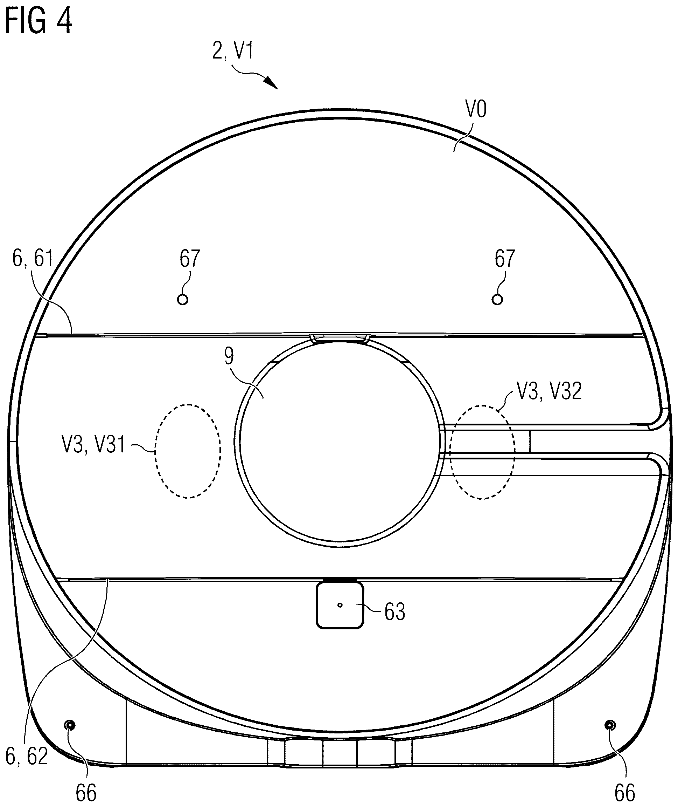

Die Figuren 1 bis 4 zeigen das Verkleidungsteil V1 für eine Gantry 20 eines Computertomographiegeräts 1 in verschiedenen Ansichten,
- wobei das Verkleidungsteil V1 ein Ringscheiben-Formteil V0 und ein Zylindermantel-Formteil V9 aufweist,
- wobei das Ringscheiben-Formteil V0 und das Zylindermantel-Formteil V9 derart koaxial zu einer Systemachse 1A angeordnet sind, dass ein Innenumfang des Ringscheiben-Formteils V0 an eine Basis des Zylindermantel-Formteils V9 angefügt ist,
- wobei das Ringscheiben-Formteil V0 einen ersten Wandungsbereich V31 und einen zweiten Wandungsbereich V32 aufweist, die in Bezug auf die Systemachse 1A einander gegenüberliegen, an den Innenumfang des Ringscheiben-Formteils V0 angrenzen und sich im Wesentlichen senkrecht zu der Systemachse 1A flächig erstrecken,
- wobei das Ringscheiben-Formteil V0 eine Versteifungsstruktur 6 aufweist, welche sich derart im Wesentlichen parallel zu der Systemachse 1A flächig von dem Zylindermantel-Formteil V9 weg erstreckt, dass sich der erste Wandungsbereich V31 und der zweite Wandungsbereich V32 in Bezug auf die Systemachse 1A zwischen der Versteifungsstruktur 6 und dem Zylindermantel-Formteil V9 befinden.

An dem Außenumfang des Ringscheiben-Formteils V0 sind Blöcke 64 befestigt, insbesondere geklebt, an die ein weiteres Teil V2 der Verkleidung V formschlüssig angefügt werden kann. Mittels der Blöcke 64 können die mechanischen Spannungen, welche aus der Verbindung des Verkleidungsteils V1 mit dem weiteren Teil V2 der Verkleidung V resultieren, über das Ringscheiben-Formteil gleichmäßig verteilt werden. Mittels der Befestigungsmittel 65, 66 kann das Verkleidungsteil V1 an dem weiteren Teil V2 der Verkleidung befestigt werden. An dem Verkleidungsteil V1 ist außerdem eine Halterung 67 für eine Haltestruktur 7 einer Strahlenschutzvorrichtung 75 befestigt. An dem Verkleidungsteil V1 ist außerdem ein Verbindungselement 63 für eine Verbindung, insbesondere lösbare elektromagnetische Verbindung, des Verkleidungsteils V1 mit einer Haltesäule 32, an der auch die Oberkörper-Lagerungsplatte 15 und die Kopfschale 19 angeordnet sind, befestigt. Das Verkleidungsteil V1 bildet zusammen mit den Bauteilen 63, 64, 65, 66, 67 und 68 eine integrierte Verkleidungs- und Haltestruktur 2 der Gantry 20.

Das gezeigte Beispiel sieht vor, dass die Versteifungsstruktur 6 eine erste Teilstruktur 61 aufweist, wobei sich die erste Teilstruktur 61 in einer ersten Versteifungsebene, die zu der Systemachse 1A im Wesentlichen parallel ist, flächig erstreckt.

Das gezeigte Beispiel sieht vor, dass die Versteifungsstruktur 6 eine zweite Teilstruktur 62 aufweist, wobei sich die zweite Teilstruktur 62 in einer zweiten Versteifungsebene, die zu der ersten Versteifungsebene parallel ist, flächig erstreckt, wobei sich der erste Wandungsbereich V31 und der zweite Wandungsbereich V32 in Bezug auf eine Richtung y, die zu der Systemachse 1A senkrecht ist, zwischen der ersten Versteifungsebene und der zweiten Versteifungsebene befinden.

Das gezeigte Beispiel sieht vor, dass das Ringscheiben-Formteil V0 und/oder das Zylindermantel-Formteil V9 aus einem faserverstärkten Verbundstoff, insbesondere einem carbonfaserverstärkten Verbundstoff, hergestellt ist.

Die Trennwand V3 weist den ersten Wandungsbereich V31 und den zweiten Wandungsbereich V32 auf. Auf die Trennwand V3 können durch einen Formschluss der Schultern des Patienten mit dem ersten Wandungsbereich V31 und dem zweiten Wandungsbereich V32 beim Einführen des Kopfes des Patienten in die Öffnung Belastungen wirken. Ähnliche Belastungen können auftreten, wenn eine Bedienperson sich an die Trennwand V3 anlehnt. Die Versteifungsstruktur 6 wirkt einem Verbiegen der Trennwand V3 unter solchen Belastungen entgegen, sodass die Trennwand V3 dünnwandig ausgebildet werden kann und/oder ein geringer Sicherheitsabstand zu den rotierenden Komponenten vorgesehen werden kann, um die Scantiefe zu erhöhen.

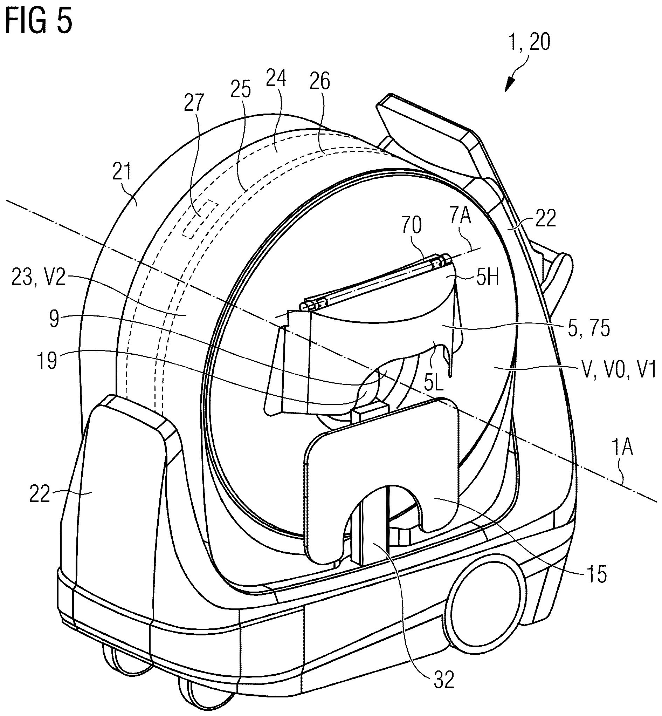

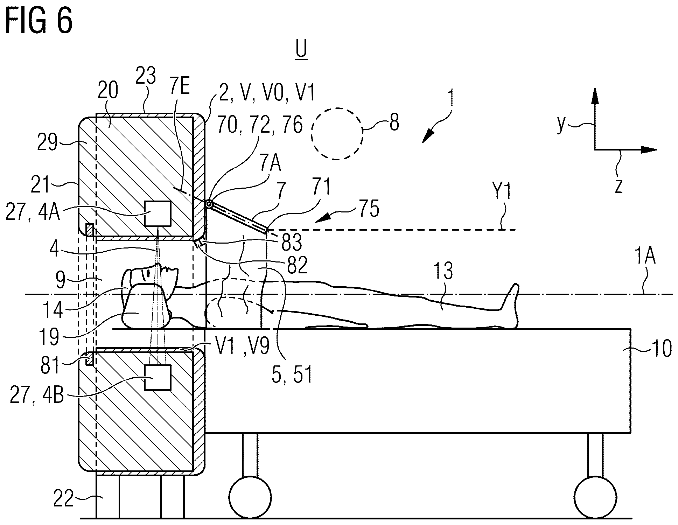

Die Figuren 5 und 6 zeigen das Computertomographiegerät 1 mit der Gantry 20 und einer Strahlenschutzvorrichtung 75 zur Abdeckung der Öffnung 9. Das gezeigte Beispiel sieht vor, dass die Gantry 20 eine Verkleidung V zum Abgrenzen eines Innenbereichs 29 der Gantry 20 von einer Umgebung U der Gantry 20 aufweist, wobei die Verkleidung V das Verkleidungsteil V1 nach dem gezeigten Beispiel aufweist, wobei die Gantry 20 eine Öffnung 9 aufweist, wobei ein Untersuchungsobjekt 14 entlang der Systemachse 1A in die Öffnung 9 einführbar ist, wobei das Zylindermantel-Formteil V9 den Innenbereich 29 der Gantry 20 von der Öffnung 9 abgrenzt.

Das gezeigte Beispiel sieht vor, dass ein Strahlenschutzmaterial an einer dem Innenbereich 29 der Gantry 20 zugewandten Seite des Ringscheiben-Formteils V0 flächig anliegt.

Das gezeigte Beispiel sieht vor, dass die Gantry 20 ein Projektionsdatenakquisitionssystem 27 aufweist, wobei das Projektionsdatenakquisitionssystem 27 zum Erfassen von Projektionsdaten des in die Öffnung 9 eingeführten Untersuchungsobjekts 14 eingerichtet ist, wobei ein Abstand der Versteifungsstruktur 6 zu der Systemachse 1A, insbesondere ein Abstand der ersten Versteifungsebene und/oder der zweiten Versteifungsebene zu der Systemachse 1A, kleiner ist als ein Abstand des Projektionsdatenakquisitionssystems 27 von der Systemachse 1A.

Das gezeigte Beispiel sieht vor, dass die Versteifungsstruktur 6 ein Fenster 60 derart aufweist, dass eine optische Achse einer optischen Komponente des Computertomographiesystems 1 durch das Fenster 60 dringen kann, wenn sich die optische Komponente in dem Innenbereich der Gantry 20 befindet.

Das gezeigte Beispiel sieht vor, dass das Verkleidungsteil V1 eine hutzenartige Wölbung hin zu der Umgebung U der Gantry 20 aufweist, wobei sich die hutzenartige Wölbung zu der Versteifungsstruktur 6, insbesondere zu der ersten Teilstruktur 61 der Versteifungsstruktur 6, hin aufweitet, wobei ein Teilbereich des Innenbereichs der Gantry 20 durch die hutzenartige Wölbung und die Versteifungsstruktur 6 von der Umgebung U der Gantry 20 abgegrenzt ist, wobei sich in dem Teilbereich des Innenbereichs der Gantry 20 eine Halterung 68 zur Befestigung der optischen Komponente an der Versteifungsstruktur 6 befindet.

Das Untersuchungsobjekt 14 ist der Kopf des Patienten 13. Das Computertomographiegerät 1 ist ein mobiles Kopf-Computertomographiegerät. Das Computertomographiegerät 1 weist ferner eine Kopfschale 19, in welche der Kopf des Patienten 13 aufgenommen werden kann, und eine Oberkörper-Lagerungsplatte 15, auf welcher der Oberkörper des Patienten 13 gelagert werden kann, auf.

Die Gantry 20 weist einen ersten Gantryteil 21, einen zweiten Gantryteil 22 und einen dritten Gantryteil 23 auf, wobei der erste Gantryteil 21 einen drehbar gelagerten Rotor 24 mit einem Projektionsdatenakquisitionssystem 27 aufweist, wobei der dritte Gantryteil 23 zumindest einen Abschnitt der Öffnung 9 aufweist. Die Haltestruktur 7 ist mittels der Schwenkvorrichtung 70 mit dem dritten Gantryteil 23 verbunden und relativ zu dem dritten Gantryteil 23 um die Schwenkachse 7A schwenkbar gelagert. Der dritte Gantryteil 23 weist das Verkleidungsteil V1 auf.

Der erste Gantryteil 21 ist relativ zu dem zweiten Gantryteil 22 und relativ zu dem dritten Gantryteil 23 derart bewegbar gelagert, dass eine Translationsbewegung des ersten Gantryteils 21 relativ zu dem zweiten Gantryteil 22 und dem dritten Gantryteil 23 ausgeführt werden kann, während gleichzeitig dazu der zweite Gantryteil 22 und der dritte Gantryteil 23 relativ zu dem Untersuchungsobjekt 14 ruhen und die Strahlenschutzvorrichtung 75 relativ zu dem Untersuchungsobjekt 14 und relativ zu dem zumindest einen Abschnitt der Öffnung 9 ruht, wenn sich das Untersuchungsobjekt 14 in der Öffnung 9 befindet.

Der erste Gantryteil 21 weist eine Drehlagerung 25 und eine Tragstruktur 26 auf, wobei der Rotor 24 mittels der Drehlagerung 25 mit der Tragstruktur 26 verbunden und relativ zu der Tragstruktur 26 um die Systemachse 1A drehbar gelagert ist. Das Projektionsdatenakquisitionssystem 27 weist die Röntgenquelle 4A zum Erzeugen der Röntgenstrahlung 4 und den Röntgendetektor 4B zum Detektieren der Röntgenstrahlung 4 auf. Das Zylindermantel-Formteil V9 befindet sich im Strahlengang der Röntgenstrahlung 4.

Die Fig. 6 zeigt das Computertomographiegerät 1 mit der Gantry 20. Das Computertomographiegerät 1 weist das Patientenbett 10 zur Lagerung des Patienten 13 auf. Die Gantry 20 weist einen Innenbereich 29 und eine Verkleidung V zum Abgrenzen des Innenbereichs 29 von einer Umgebung U auf. Das gezeigte Beispiel sieht vor, dass der obere Bereich 5H des Strahlenschutzvorhangs 5 auf der Haltestruktur 7 derart aufliegt, dass der obere Bereich 5H des Strahlenschutzvorhangs 5 einen Bereich 8 einer Umgebung U der Gantry 20 vor Streustrahlen einer Röntgenstrahlung 4, die aus der Öffnung 9 kommend auf den Bereich 8 der Umgebung U der Gantry 20 gerichtet sind und die Haltestruktur 7 durchdringen, abschirmt, insbesondere wenn sich die Haltestruktur 7 in der gezeigten Position der Haltestruktur 7 befindet.

Die Haltestruktur 7 erstreckt sich flächig in einer Deckelebene 7E, wobei die Deckelebene 7E zu der Schwenkachse 7A im Wesentlichen parallel ist. Die Systemachse 1A ist horizontal und parallel zu der horizontalen Richtung z. Die Schwenkachse 7A ist horizontal und senkrecht zu der horizontalen Richtung z. Die Schwenkachse 7A befindet sich oberhalb der Öffnung 9. Der Strahlenschutzvorhang 5 ist an der Haltestruktur 7 angeordnet, insbesondere an der Haltestruktur 7 befestigt, wobei ein unterer Bereich 5L des Strahlenschutzvorhangs 5 von der Haltestruktur 7 herabhängt, wobei ein oberer Bereich 5H des Strahlenschutzvorhangs 5 auf der Haltestruktur 7 aufliegt. Die Schwenkvorrichtung 70 weist das Friktionsscharnier 76 auf. Ein erster seitlicher Teil 51 des Strahlenschutzvorhangs 5 erstreckt sich von der Gantry 20 bis zu einem ersten Rand 71 der Haltestruktur 7. Y1 ist die vertikale Position des ersten Rands 71 der Haltestruktur 7. In Bezug auf die vertikale Richtung y befindet sich ein Zwischenraum zwischen dem Patienten 13 und der Haltestruktur 7. Dadurch ist die Gefahr, dass der Patient 13 mit der Haltestruktur 7 kollidiert, verringert. Das Computertomographiegerät 1 weist ferner ein Beleuchtungssystem 81 auf, welches dazu eingerichtet ist, die Öffnung 9 zu beleuchten.

Das Computertomographiegerät 1 weist ferner eine Kamera 82 auf, welche dazu eingerichtet ist, das Untersuchungsobjekt 14 zumindest dann optisch zu erfassen, wenn sich das Untersuchungsobjekt 14 in der Öffnung 9 und/oder unmittelbar vor der Öffnung 9 befindet. Das Computertomographiegerät 1 weist ferner eine Lasereinheit 83 zur laserbasierten Positionierung des Untersuchungsobjekts 14 auf.

## Patentansprüche

1. Verkleidungsteil (V1) für eine Gantry (20) eines Computertomographiegeräts (1),
- wobei das Verkleidungsteil (V1) ein Ringscheiben-Formteil (V0) und ein Zylindermantel-Formteil (V9) aufweist,
- wobei das Ringscheiben-Formteil (V0) und das Zylindermantel-Formteil (V9) derart koaxial zu einer Systemachse (1A) angeordnet sind, dass ein Innenumfang des Ringscheiben-Formteils (V0) an eine Basis des Zylindermantel-Formteils (V9) angefügt ist,
- wobei das Ringscheiben-Formteil (V0) einen ersten Wandungsbereich (V31) und einen zweiten Wandungsbereich (V32) aufweist, die in Bezug auf die Systemachse (1A) einander gegenüberliegen, an den Innenumfang des Ringscheiben-Formteils (V0) angrenzen und sich im Wesentlichen senkrecht zu der Systemachse (1A) flächig erstrecken,
- wobei das Ringscheiben-Formteil (V0) eine Versteifungsstruktur (6) aufweist, welche sich derart im Wesentlichen parallel zu der Systemachse (1A) flächig von dem Zylindermantel-Formteil (V9) weg erstreckt, dass sich der erste Wandungsbereich (V31) und der zweite Wandungsbereich (V32) in Bezug auf die Systemachse (1A) zwischen der Versteifungsstruktur (6) und dem Zylindermantel-Formteil (V9) befinden.

2. Verkleidungsteil (V1) nach Anspruch 1,
- wobei die Versteifungsstruktur (6) eine erste Teilstruktur (61) aufweist,
- wobei sich die erste Teilstruktur (61) in einer ersten Versteifungsebene, die zu der Systemachse (1A) im Wesentlichen parallel ist, flächig erstreckt.

3. Verkleidungsteil (V1) nach Anspruch 2,
- wobei die Versteifungsstruktur (6) eine zweite Teilstruktur (62) aufweist,
- wobei sich die zweite Teilstruktur (62) in einer zweiten Versteifungsebene, die zu der ersten Versteifungsebene parallel ist, flächig erstreckt,
- wobei sich der erste Wandungsbereich (V31) und der zweite Wandungsbereich (V32) in Bezug auf eine Richtung (y), die zu der Systemachse (1A) senkrecht ist, zwischen der ersten Versteifungsebene und der zweiten Versteifungsebene befinden.

4. Verkleidungsteil (V1) nach einem der Ansprüche 1 bis 3,
- wobei das Ringscheiben-Formteil (V0) und/oder das Zylindermantel-Formteil (V9) aus einem faserverstärkten Verbundstoff, insbesondere einem carbonfaserverstärkten Verbundstoff, hergestellt ist.

5. Gantry (20) für ein Computertomographiegerät (1),
- wobei die Gantry (20) eine Verkleidung (V) zum Abgrenzen eines Innenbereichs (29) der Gantry (20) von einer Umgebung (U) der Gantry (20) aufweist,
- wobei die Verkleidung (V) das Verkleidungsteil (V1) nach einem der Ansprüche 1 bis 4 aufweist,
- wobei die Gantry (20) eine Öffnung (9) aufweist, wobei ein Untersuchungsobjekt (14) entlang der Systemachse (1A) in die Öffnung (9) einführbar ist,
- wobei das Zylindermantel-Formteil (V9) den Innenbereich (29) der Gantry (20) von der Öffnung (9) abgrenzt.

6. Gantry (20) nach Anspruch 5,
- wobei ein Strahlenschutzmaterial an einer dem Innenbereich (29) der Gantry (20) zugewandten Seite des Ringscheiben-Formteils (V0) flächig anliegt.

7. Gantry (20) nach Anspruch 5 oder 6,
- wobei die Gantry (20) ein Projektionsdatenakquisitionssystem (27) aufweist,
- wobei das Projektionsdatenakquisitionssystem (27) zum Erfassen von Projektionsdaten des in die Öffnung (9) eingeführten Untersuchungsobjekts (14) eingerichtet ist,
- wobei ein Abstand der Versteifungsstruktur (6) zu der Systemachse (1A), insbesondere ein Abstand der ersten Versteifungsebene und/oder der zweiten Versteifungsebene zu der Systemachse (1A), kleiner ist als ein Abstand des Projektionsdatenakquisitionssystems (27) von der Systemachse (1A).

8. Gantry (20) nach einem der Ansprüche 5 bis 7,
- wobei die Versteifungsstruktur (6) ein Fenster (60) derart aufweist, dass eine optische Achse einer optischen Komponente des Computertomographiesystems (1) durch das Fenster (60) dringen kann, wenn sich die optische Komponente in dem Innenbereich der Gantry (20) befindet.

9. Gantry (20) nach Anspruch 8,
- wobei das Verkleidungsteil (V1) eine hutzenartige Wölbung hin zu der Umgebung (U) der Gantry (20) aufweist,
- wobei sich die hutzenartige Wölbung zu der Versteifungsstruktur (6), insbesondere zu der ersten Teilstruktur (61) der Versteifungsstruktur (6), hin aufweitet,
- wobei ein Teilbereich des Innenbereichs der Gantry (20) durch die hutzenartige Wölbung und die Versteifungsstruktur (6) von der Umgebung (U) der Gantry (20) abgegrenzt ist,
- wobei sich in dem Teilbereich des Innenbereichs der Gantry (20) eine Halterung (68) zur Befestigung der optischen Komponente an der Versteifungsstruktur (6) befindet.

10. Computertomographiegerät (1), insbesondere ein mobiles Kopf-Computertomographiegerät, aufweisend eine Gantry (20) nach einem der Ansprüche 5 bis 9.
